**Europäisches Patentamt**

(19) **European Patent Office**

**Office européen des brevets**

(11) Veröffentlichungsnummer: **0 011 243**
**B1**

(12) **EUROPÄISCHE PATENTSCHRIFT**

(45) Veröffentlichungstag der Patentschrift:
14.04.82

(51) Int. Cl.³: **C 12 P 21/00**, A 61 K 39/095 //
C12R1/36

(21) Anmeldenummer: **79104391.2**

(22) Anmeldetag: **08.11.79**

(54) Verfahren zur Herstellung von Membranproteinen aus Neisseria meningitidis und diese enthaltende Vaccine.

(30) Priorität: **11.11.78 DE 2848965**

(43) Veröffentlichungstag der Anmeldung:
**28.05.80 Patentblatt 80/11**

(45) Bekanntmachung des Hinweises auf die Patenterteilung:
**14.04.82 Patentblatt 82/15**

(84) Benannte Vertragsstaaten:
**AT BE CH DE FR GB IT NL SE**

(73) Patentinhaber: **BEHRINGWERKE
AKTIENGESELLSCHAFT, Postfach 1140,
D-3550 Marburg/Lahn (DE)**

(72) Erfinder: **Helting, Torsten Bertil, Dr., Oberer Eichweg 24,
D-3550 Marburg/Lahn (DE)**
Erfinder: **Guthöhrlein, Gerhard, Dr., Am Ziegenberg 8,
D-3550 Marburg/Lahn (DE)**

(74) Vertreter: **Meyer-Dulheuer, Karl-Hermann, Dr. et al,
HOECHST Aktiengesellschaft Zentrale Patentabteilung
Postfach 80 03 20, D-6230 Frankfurt/Main 80 (DE)**

(56) Entgegenhaltungen:

CHEMICAL ABSTRACTS, Band 64, Nr. 13,
20. Juni 1966, Zusammenfassung Nr. 20236e,
Columbus, Ohio, US,
N.A. VEDROS et al.: «Chemical and antigenic analysis
of the cell walls of Neisseria meningitidis group B»

CHEMICAL ABSTRACTS, Band 89, Nr. 15,
9. Oktober 1978, Zusammenfassung Nr. 127600p.
Seite 441,
Columbus, Ohio, US,
W.D. ZOLLINGER et al.: «Safety and immunogenicity
of Neisseria meningitidis type 2 protein vaccine in
animals and humans»

(56) Entgegenhaltungen:

THE JOURNAL OF EXPERIMENTAL MEDICINE,
Band 147, 1978, Nr. 3,
C.E. FRASCH et al.: «Protection against group B meningococcal disease III. Immunogenicity of serotype 2
vaccines and specificity of protection in a guinea pig
model», Seiten 629-644

Verfahren zur Herstellung von Membranproteinen aus Neisseria meningitidis und diese enthaltende Vaccine

Die Erfindung betrifft ein verbessertes Verfahren zur Herstellung von Membranproteinen aus Neisseria meningitidis sowie eine diese Membranproteine enthaltende Vaccine.

Der Erreger der Meningitis cerebrospinalis epidemica ist die Neisseria meningitidis, auch Meningokokkus genannt. Die durch Tröpfcheninfektion übertragbare Genickstarre oder Hirnhautentzündung tritt vor allem bei Kindern und Personen in Massenquartieren auf. Es besteht deshalb ein Bedürfnis, gefährdete Personengruppen zu immunisieren.

Nach vorliegenden Erkenntnissen ist das wichtigste Membranprotein (Major outer Membrane protein) der Neisseria meningitidis in der Lage, bakterizide Antikörper zu induzieren. Diese bieten einen serologisch typenspezifischen Schutz gegen die Erkrankung. Andere Komponenten der Neisserien, insbesondere die Kapselpolysaccharide der Neisserien Gruppe A und C können einen gruppenspezifischen Schutz bewerkstelligen. Sie induzieren jedoch nicht einen wirksamen Schutz gegen Neisserien der Gruppe B. Auch fehlen Hinweise dafür, dass das Kapselpolysaccharid der Gruppe B einen solchen Schutz induzieren kann. Deshalb sind die Membranproteine der Neisseria meningitidis Gruppe B von besonderem Interesse.

Für die Herstellung einer Vaccine ist es erforderlich, Membranprotein aus Neisseria meningitidis Gruppe B in ausreichender Menge zu gewinnen. Das von Frasch in J. Bact. 127, 973 - 981 (1976) beschriebene Verfahren führt zu einem brauchbaren Präparat. Die Ausbeute nach diesem Verfahren liegt jedoch an der unteren, wirtschaftlich vertretbaren Grenze. Das Wesen des Verfahren nach Frasch besteht darin, dass Keime der Neisseria meningitidis mit Salzlösungen (CaCl$_2$- oder LiCl-Lösungen) zunächst gewaschen und der Extrakt anschliessend mit Desoxycholat behandelt wird. Wie bereits erwähnt, führt dieses Verfahren jedoch zu einer schlechten Ausbeute.

Es wurde nun überraschend gefunden, dass die Ausbeute wesentlich erhöht werden kann, wenn die Keimmasse einer direkten Behandlung mit Detergenzien unterzogen wird. Das erfindungsgemäss gewonnene Material unterscheidet sich nicht erkennbar von Membranproteinpräparaten nach Frasch. Es besitzt die gleichen elektrophoretischen Eigenschaften in SDS-haltigen Pufferlösungen. Sein Endotoxingehalt ist mit dem nach Frasch erhaltenen Material ebenso vergleichbar wie seine Fähigkeit, schützende Antikörper in Tieren zu induzieren.

Gegenstand der Erfindung ist demnach ein Verfahren zur Herstellung von Membranprotein aus Neisseria meningitidis, insbesondere solcher der Gruppe B, dadurch gekennzeichnet, dass man diese mit der wässrigen Lösung eines Detergenz in einer Konzentration von 0,1% bis 2% für 15 Minuten bis 24 Stunden, ggf. unter Bewegung, stehen lässt, den Extrakt vom Bakterienrückstand abtrennt und gegebenenfalls weiter reinigt.

Als Detergenz wird bevorzugt Desoxycholat, insbesondere in seiner relativ leicht wasserlöslichen Form des Natriumsalzes verwendet. Die Konzentration des Detergenz von 0,1 bis 2% bezieht sich auf das Gewicht des Detergenz in Gramm pro Volumen in ml. Das Verhältnis von feuchter Bakterienmasse zur Detergenzienlösung liegt vorteilhaft bei 1 : 3 - 1 : 20, vorzugsweise 1 : 5 (w/v).

Sofern als Detergenz Natriumdesoxycholat verwendet wird, wird dieses in einer Konzentration von 0,3% bis 1% in einer wässrigen Lösung verwendet. Als wässrige Lösungen kommen vor allem die in der biochemischen oder mikrobiologischen Praxis üblichen Pufferlösungen in Frage.

Als Ausgangsmaterial wird die nach bekannten Verfahren erhältliche Kultur der Neisseria meningitidis gewonnen und die Zellmasse vom Nährbodenüberstand abgetrennt. Vorteilhaft ist hierfür die Zentrifugation. Der Zentrifugenrückstand wird in der detergenzhaltigen Lösung suspendiert und erfindungsgemäss eine Zeitlang stehen gelassen. Danach wird der Extrakt von dem Zellrückstand abgetrennt. Vorteilhaft ist hier ebenfalls die Durchführung einer Zentrifugation.

Die Temperatur, bei der die Extraktion ausgeführt wird, wird nach unten durch den Gefrierpunkt der verwendeten Lösungen begrenzt. Die obere Grenze ergibt sich durch den Verlust der Immunogenität der Membranproteine durch Hitzedenaturierung. Daher empfiehlt es sich, im Temperaturbereich zwischen Raumtemperatur und etwa 60°C zu arbeiten.

Der Extrakt kann gewünschtenfalls weiter gereinigt werden. Es ist zweckmässig, hierzu zunächst den bei Ultrazentrifugation gewinnbaren Rückstand einer nochmaligen Extraktion mit der detergenzhaltigen Lösung zu unterwerfen und diese Extraktion gewünschtenfalls nochmals zu wiederholen. Dabei wird jeweils der Ultrazentrifugationsüberstand verworfen und der Rückstand in der detergenzhaltigen wässrigen Lösung suspendiert.

Eine Anreicherung bzw. Reinigung ist auch mit in der Biochemie üblichen Fällungsmitteln für Proteine und entsprechende Antigene möglich. So kann das Neisseria meningitidis-Antigen durch Zusatz von beispielsweise 4 Volumenteilen Äthanol gefällt und die Fällung in wässriger Lösung wiederaufgenommen werden.

Das steril filtrierte und evtl. gefriergetrocknete Neisseria meningitidis-Antigen ist geeignet, schützende Antikörper gegen den Erreger zu induzieren.

Dieses Antigen kann mit Adjuvantien, stabilisierenden Zusätzen, Füllstoffen und ähnlichen Substanzen, wie sie bei der Vaccineherstellung üblicherweise Anwendung finden, versetzt werden.

Die Membranproteine entfalten eine immunogene Wirkung. Eine die erfindungsgemäss herstellbaren Membranproteine in einer immunisatorisch wirksamen Menge enthaltende Vaccine stellt im besonderen den Gegenstand der Erfindung dar. Die Vaccine kann in einer Dosierung von etwa 10 - 200 µg pro Dosis verabreicht werden.

Die Erfindung soll an nachstehenden Beispielen näher erläutert werden:

Beispiel 1

300 g Keimmasse (Nassgewicht) von Neisseria meningitidis Gruppe B, Stamm 986, wurden in 1'500 ml Natriumdesoxycholatlösung (0,5% Natriumdesoxycholat in 0,01 M Tris-HCl, pH 8,5, 0,01 M EDTA), die vorher auf 60°C gebracht worden war, resuspendiert und 15 Minuten in einem Wasserbad (56°C) gehalten. Anschliessend erfolgte eine Zentrifugation zur Abtrennung der Keimmasse (Sorvall GSA Rotor, 10 000 U/Min.). Der Rückstand wurde anschliessend wie oben nochmals mit Natriumdesoxycholatlösung 15 Min. behandelt und der zweite Überstand nach erneuter Zentrifugation mit dem ersten Überstand vereinigt. Die vereinigten Überstände wurden dann in einer Beckman L 75 Zentrifuge bei 40 000 U/Min. in einem 45 Ti Rotor 60 Min. geschleudert. Das Sediment wurde in 150 ml Natriumdesoxycholatlösung suspendiert und über Nacht bei 4°C gerührt.

Zur Weiterreinigung wurde die Suspension am nächsten Tag in einem Wasserbad (56°C, 15 Min.) erhitzt, erneut in der Ultrazentrifuge wie oben (Rotor 45 Ti 40 000 U/Min., 60 Min.) geschleudert und das Sediment in 150 ml Natriumdesoxycholatlösung resuspendiert. Zur Resuspension wurde ein Ultrasonic Cleaner der Firma Laboratory Supplies Co., Inc, Hicksville N.Y., USA, verwendet. Aliquote à 50 ml wurden jeweils 3 Minuten beschallt und anschliessend vereinigt. Partikuläres Material wurde durch eine abermalige Zentrifugation (Sorvall, SS-34 Rotor 20 000 U/Min., 20 Min.) abgetrennt und der Überstand filtriert. Das Membranprotein wurde anschliessend durch Zusatz von 600 ml 96%igem Äthanol steril ausgefällt und 60 Min. stehengelassen. Die Fällung wurde durch Zentrifugation (Sorvall, GSA Rotor) gewonnen, einmal mit 100 ml Äthanol gewaschen und anschliessend in 150 ml 5%iger Raffinose steril aufgenommen und über Nacht bei 4°C gerührt.

Die optische Dichte (O.D.) des Materials (280 nm) wurde bestimmt und durch Zusatz von 5%iger Raffinose auf O.D.$_{280}$ = 1,2 eingestellt. Dieses Konzentrat wurde dann in üblicher Weise zu einer Vaccine verarbeitet. Zur Bestimmung der optischen Dichte wurde ein aliquoter Teil unter sterilen Bedingungen entnommen, mit 1 Vol. Trichloressigsäure (TCA) (10%ig) gefällt, die Fällung in 5%iger TCA gewaschen und in 1 M KOH aufgenommen.

Beispiel 2

50 g Keimmasse wurde wie in Beispiel 1 aufgearbeitet, aber statt 0,5% Natriumdesoxycholat wurde eine Lösung mit 0,1% Natriumdesoxycholat verwendet. Die Ausbeute betrug 0,8 mg/g Zellmasse, während die Ausbeute nach Ausführungsbeispiel 1 2,3 mg/g betrug.

Beispiel 3

50 g Keimmasse wurde wie in Beispiel 1 aufgearbeitet, aber statt 0,5% Natriumdesoxycholat wurde eine Lösung mit 2,0% Natriumdesoxycholat verwendet. Die Ausbeute betrug 2,2 mg/g Zellmasse, während die Ausbeute nach Ausführungsbeispiel 1 2,3 mg/g betrug.

Beispiel 4

50 g Keimmasse wurde wie in Beispiel 1 aufgearbeitet, aber statt 0,5% Natriumdesoxycholat in 0,01 M Tris-HCl-0,01 M EDTA wurde 0,5% Natriumdesoxycholat in a) 0,1 M Phosphatpuffer, pH 7,8, b) Hepes Puffer (0,1 M, pH 7,8) oder c) 0,1 M Natriumcarbonatpuffer, pH 8,5, verwendet. Die Ausbeuten variieren zwischen 1,8 mg/g und 2,5 mg/g.

Beispiel 5

Entsprechend Beispiel 1 wurde die Extraktion mit weiteren Detergenzien ausgeführt. Die Ausbeuten zeigt folgende Tabelle:

| Detergenz | Konzentration | Ausbeute (mg/g Nassgewicht) |
|---|---|---|
| Harnstoff | 4 M | 1,2 |
| Emulprogen 1% (GAF Corp.) | 1% | 1,3 |
| Tween 20 | 1% | 2,6 |
| Triton X-100 | 1% | 2,7 |

| | |
|---|---|
| Tris-HCL | = Tris-(hydroxymethyl)amino-methan-hydrochlorid |
| EDTA | = Ethylendiamintetraacetat |
| Emulphogene® | BC-720 (GAF Corp.) = Polyoxyethylen(10)-tridecylether |
| Tween® 20 | (Atlas Chemical Industries, Inc) = Polyoxyethylen(20)-sorbitanmonolaurat |
| Triton® X-100 | (Rohm and Haas Corp.) = Polyoxyethylen(10)-p-isooctyl-phenylether |

**Patentansprüche**

1. Verfahren zur Herstellung von Membranproteinen aus Neisseria meningitidis, dadurch gekennzeichnet, dass man diese mit der wässrigen Lösung eines Detergenz in einer Konzentration von 0,1 bis 2% w/v für 15 Minuten bis 24 Stunden, gegebenenfalls unter Bewegung, stehenlässt, den Extrakt vom Bakterienrückstand abtrennt und ggf. weiter reinigt.

2. Verfahren nach Anspruch 1, dadurch gekennzeichnet, dass als Neisseria meningitidis ein Stamm der Gruppe B verwendet wird.

3. Verfahren nach Anspruch 1, dadurch gekennzeichnet, dass als Detergenz Desoxycholat verwendet wird.

4. Vaccine gegen Meningitis cerebrospinalis epidemica, gekennzeichnet durch den Gehalt einer wirksamen immunisierenden Menge eines nach dem Verfahren der Ansprüche 1, 2 oder 3 erhältlichen Membranproteins.

**Claims**

1. Process for the isolation of membrane proteins from Neisseria meningitidis which comprises treating the organism with the aqueous solution of a detergent in a concentration of 0.1 to 2% w/v for 15 minutes to 24 hours, allowing the mixture to stand, optionally with agitation, separating the extract from the bacterial residue and optionally carrying out a further purification.

2. The process of claim 1, wherein as Neisseria meningitidis a group B strain is used.

3. The process of claim 1, wherein the detergent is desoxycholate.

4. Vaccine against Meningitidis cerebrospinalis epidemica containing an amount sufficient for immunization of the membrane proteins obtained by the process claimed in claims 1, 2 or 3.

**Revendications**

1. Procédé de préparation de protéines membranaires à partir de Neisseria méningitidis, caractérisé en ce qu'il consiste à mettre celle-ci en contact avec la solution aqueuse d'un détergent en une concentration de 0,1 à 2% en poids/ volume pendant 15 minutes à 24 heures, éventuellement en agitant, puis à séparer l'extrait du résidu bactérien et éveutuellement à purifier de nouveau.

2. Procédé selon la revendication 1, caractérisé en ce que comme Neisseria meningitidis, on utilise une souche du groupe B.

3. Procédé selon la revendication 1, caractérisé en ce que comme détergent, on utilise un désoxycholate.

4. Vaccin contre la méningite cérébrospinale épidémique, caractérisé en ce qu'il contient une quantité immunisante efficace d'une protéine membranaire obtenue selon le procédé des revendications 1, 2 ou 3.